(19) Europäisches Patentamt
European Patent Office
Office européen des brevets

(11) Numéro de publication : **0 599 749 A1**

# (12) DEMANDE DE BREVET EUROPEEN

(21) Numéro de dépôt : **93420462.9**

(22) Date de dépôt : **22.11.93**

(51) Int. Cl.$^5$ : **C07D 233/80,** C07D 235/02, C07D 401/12, A01N 43/50, C07D 409/04

(30) Priorité : **25.11.92 FR 9214432**

(43) Date de publication de la demande : **01.06.94 Bulletin 94/22**

(84) Etats contractants désignés : **AT BE CH DE DK ES FR GB GR IE IT LI LU NL PT SE**

(71) Demandeur : **RHONE-POULENC AGROCHIMIE
14-20, rue Pierre Baizet
F-69009 Lyon (FR)**

(72) Inventeur : **Bascou, Jean-Philippe
3, rue Gabriel Chevallier
F-69009 Lyon (FR)**
Inventeur : **Emeric, Gilbert
97, Chemin des Tuileries
F-69570 Dardilly (FR)**
Inventeur : **Lacroix, Guy
332 F rue du Doyen Chapas
F-69009 Lyon (FR)**
Inventeur : **Perez, Joseph
19 Allée D - rue Ernest Fabrègue
F-69009 Lyon (FR)**
Inventeur : **Pinard, Fabrice
Rés. Jardins Agro-Bât C no. 7,
36 rue Buffon
F-34000 Montpellier (FR)**

## (54) Dérivés de 2-alkoxy 2-imidazoline-5 ones fongicides.

(57) L'invention concerne des dérivé de 2-alkoxy-2-imidazoline-5-ones de formule générale (I) :

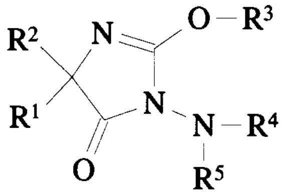

dans laquelle :
$R^1$ représente un radical aryl,
$R^2$ représente un radical alkyl ou haloalkyl,
$R^3$ représente un groupe alkyl ou haloalkyl,
$R^4$ représente un radical aryl,
$R^5$ représente H, ou un radical formyl, acyl, aroyl, alkoxycarbonyl, aryloxycarbonyl, alkylsulfonyl, arylsulfonyl ;
L'invention concerne également la préparation de ces composés et leur utilisation comme fongicides à spectre large.

EP 0 599 749 A1

La présente invention concerne de nouveaux composés 2-alkoxy-2-imidazoline-5-ones à usage phytosanitaire. Elle concerne également les procédés de préparation desdits composés et les produits éventuellement utilisables à titre d'intermédiaires dans les procédés de préparation. Elle concerne ensuite l'utilisation à titre de fongicides de ces composés, les compositions fongicides à base de ces composés et les procédés pour lutter contre les maladies fongiques des cultures utilisant ces composés.

Un but de la présente invention est de proposer des composés présentant des propriétés améliorées dans le traitement des maladies fongiques.

Un autre but de la présente invention est de proposer des composés présentant un spectre d'utilisation également amélioré dans le domaine des maladies fongiques.

Il a maintenant été trouvé que ces buts pouvaient être atteints en totalité ou en partie grâce aux produits de l'invention, qui sont des dérivés de 2-alkoxy-2-imidazoline-5-ones de formule générale (I) :

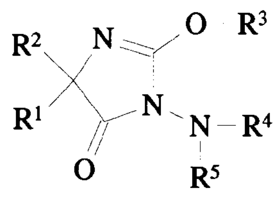

dans laquelle:

- $R^1$ représente un radical aryl comprenant phényl, naphtyl, thiényl, furyl, pyridyl, benzothiényl, benzofuryl, quinolinyl, isoquinolinyl, ou méthylène dioxyphényl, chacun de ces radicaux étant éventuellement substitué par 1 à 3 groupements choisis parmi les significations de $R^6$ ;
- $R^2$ représente un radical alkyl ou haloalkyl de 1 à 3 atomes de carbone,
- $R^1$ et $R^2$ pouvant en outre former, avec le carbone auquel ils sont liés sur le cycle, un carbocycle ou un hétérocycle ayant de 5 à 7 atomes, ces cycles pouvant être fusionnés avec un phényl, chacun de ces radicaux étant éventuellement substitué par 1 à 3 groupes choisis parmi les significations de $R^6$ ;
- $R^3$ représente un groupe alkyl ou haloalkyl de 1 à 3 atomes de carbone ;
- $R^4$ représente un radical aryl, comprenant phényl, naphtyl, pyridyl, pyrimidyl, pyridazinyl, pyrazinyl, benzothiényl, benzofuryl, quinolinyl, isoquinolinyl, ou méthylène dioxyphényl, chacun de ces radicaux étant éventuellement substitué par 1 à 3 groupements choisis parmi les significations de $R^6$ ;
- $R^5$ représente un atome d'hydrogène ou un radical formyl, acyl de 2 à 6 atomes de carbone, aroyl, alkoxycarbonyl de 2 à 6 atomes de carbone, aryloxycarbonyl, alkylsulfonyl, arylsulfonyl;
- $R^6$ représente:
  - un atome d'halogène ou
  - un radical alkyl, haloalkyl, cyanoalkyl, alkoxy, haloalkoxy, cyanoalkoxy, alkylthio, haloalkylthio, cyanoalkylthio ou alkylsulfonyl de 1 à 6 atomes de carbone ou
  - un radical cycloalkyl, halocycloalkyl, alcényl, alcynyl, alcényloxy, alcynyloxy, alcénylthio, alcynylthio de 3 à 6 atomes de carbone ou
  - le groupe nitro ou cyano ou thiocyanato ou
  - un radical amino éventuellement mono ou disubstitué par un radical alkyl ou acyl de 1 à 6 atomes de carbone ou alkoxycarbonyl de 2 à 6 atomes de carbone
  - un radical phényl, phénoxy, phénylthio, phénylsulfonyl ou pyridyloxy, ces radicaux étant éventuellement substitués par un ou plusieurs des groupes suivants :
    - un atome d'halogène ou
    - un radical alkyl, haloalkyl, cyanoalkyl, alkoxy, haloalkoxy, alkylthio, haloalkylthio ou alkylsulfonyl de 1 à 6 atomes de carbone ou
    - un radical cycloalkyl, halocycloalkyl, alcényl, alcynyl, alcényloxy, alcynyloxy, alcénylthio, alcynylthio de 3 à 6 atomes de carbone ou
    - le groupe nitro ou cyano ou thiocyanato ou
    - un radical amino éventuellement mono ou disubstitué par un radical alkyl ou acyl de 1 à 6 atomes de carbone ou alkoxycarbonyl de 2 à 6 atomes de carbone
    - un radical phényl, phénoxy, phénylthio, phénylsulfonyl ou pyridyloxy,

et leurs formes salifiées.

Les composés de formule II ci dessous représentent des composés préférés :

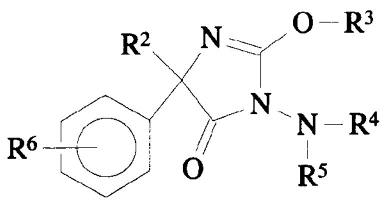

II

dans laquelle $R^1$ à $R^6$ ont les mêmes significations que précédemment.

De façon encore plus préférée, $R^4$ représente un phenyl, un phenyl substitué, un pyridyl ou un pyridyl substitué, et $R^5$ représente un atome d'hydrogène ou un radical acyle (par exemple acétyle).

Les composés de formule I sont obtenus en faisant réagir les 2alkylthio-2-imidazoline-5-ones de formule (III) avec un alcool $R^3OH$ en présence de base forte, selon le schéma :

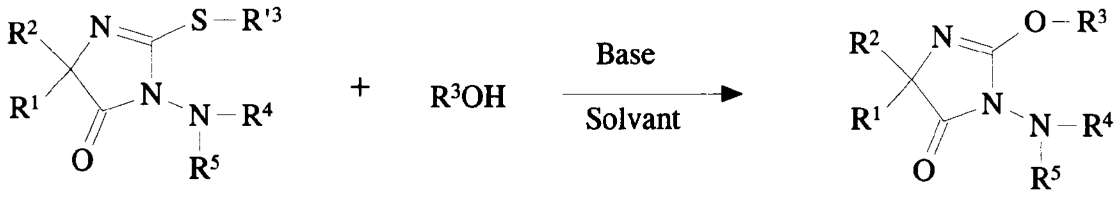


III I

$R^1$, $R^2$, $R^3$ et $R^4$ ayant la même signification que pour la formule I et $R'^3$ représentant un groupe alkyl de 1 à 3 atomes de carbone.

Comme base forte, on peut utiliser un alcoolate alcalin $R^3O^-M^+$, un hydroxyde alcalin ou une base organique forte. La réaction est pratiquée de préférence dans l'alcool $R^3OH$ comme solvant en utilisant l'alcoolate de sodium correspondant $R^3O^-Na^+$ comme base. La réaction est pratiquée à une température comprise entre 20 et 80°C. Les 2-alkylthio-2-imidazoline-5-ones de formule (III) sont obtenus selon un des procédés décrits dans la demande de brevet européen EP 0 551 048. L'acylation des composés de formule I, dans laquelle $R^5$ est l'atome d'hydrogène, se fait selon des méthodes classiques.

Les sels des composés de formule I peuvent être préparés par des méthodes connues en soi.

Les exemples ci-après sont donnés à titre d'illustration des composés selon l'invention, de leurs procédés de préparation et de leurs propriétés antifongiques.

Les structures de tous les produits ont été établies par au moins 1 des techniques spectrales suivantes: spectrométrie RMN du proton, spectrométrie RMN du carbone 13, spectrométrie Infra-Rouge et spectrométrie de masse.

Dans le tableau ci-après, les radicaux méthyl, éthyl, propyl, pyridyl, acétyl et phényl sont respectivement représentés par Me, Et, Pr, Py, Ac et Ph, et PF signifie point de fusion (PF).

Exemple 1: Préparation du composé n° 2.

Dans un ballon tricol de 100 ml, sous atmosphère d'azote sec, on fait réagir 1,4 g (1,74 mmoles) de sodium dans 60 ml d'éthanol. On ajoute ensuite 4 g (1,28 mmoles) de 4-méthyl-2-méthylthio-4-phényl-1-phénylamino-2-imidazoline-5-one. On porte à reflux pendant 6h. On refroidit le milieu à température ambiante, on acidifie avec 0,4 ml d'acide acétique. On dilue le milieu avec 300 ml d'acétate d'ethyle. On lave la solution organique à l'eau, puis on la sèche sur sulfate de magnésium puis on concentre sous pression réduite : on obtient un miel brun rouge. Le produit est purifié par chromatographie sur colonne de silice. On obtient 1,25 g (rendement de 31%) du composé n°2 sous forme d'une poudre légèrement rosée fondant à 106°C.

De la même manière, nous avons préparé les composés décrits ci-après:

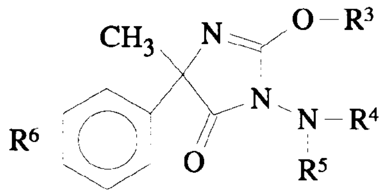

| N° | R6 | R3 | R4 | R5 | PF (°C) |
|---|---|---|---|---|---|
| 1 | - | Me | Ph | H | 149 |
| 2 | - | Et | Ph | H | 106 |
| 3 | - | nPr | Ph | H | 80 |
| 4 | 4-Cl | Me | Ph | H | 140 |
| 5 | - | Me | 2-Me-Ph | H | 118 |
| 6 | 4-Cl | Me | 2-Pyridyl | H | 150 |
| 8 | - | Me | 2-Cl-Ph | H | 94 |
| 9 | - | Me | 4-Cl-Ph | H | 124 |
| 11 | 3,4-di(MeO) | Me | Ph | H | 150 |
| 12 | 4-Cl | Me | 4-Cl-Ph | H | 176 |
| 13 | 4-Cl | Me | 4-F-Ph | H | 147 |
| 14 | 4-Cl | Me | 2-Cl-Ph | H | miel |
| 15 | 4-Cl | Me | 3-Cl-Ph | H | 147 |
| 16 | 4-Cl | Me | 4-Me-Ph | H | 162 |
| 17 | 4-Cl | Me | 3-Me-Ph | H | 116 |
| 18 | 4-Me | Me | Ph | H | 110 |
| 19 | 4-F | Me | Ph | H | 104 |

| | | | | | |
|---|---|---|---|---|---|
| 20 | 4-Cl | Me | 2-Me-Ph | H | miel |
| 21 | 4-F | Me | 3-Me-Ph | H | 114 |
| 23 | 4-F | Me | 2-Me-Ph | H | 88 |
| 24 | 4-Me | Me | 4-Cl-Ph | H | 166 |
| 25 | 4-Me | Me | 3-Cl-Ph | H | 155 |
| 27 | 4-Me | Me | 2-Cl-Ph | H | 86 |
| 28 | 4-Me | Me | 4-F-Ph | H | 124 |
| 29 | 4-F | Me | 3-Cl-Ph | H | 160 |
| 30 | 4-Me | Me | 4-Me-Ph | H | 155 |
| 31 | 4-F | Me | 4-Me-Ph | H | 149 |
| 32 | 4-Me | Me | 2-Me-Ph | H | 128 |
| 33 | - | Me | 3-F-Ph | H | 121 |
| 34 | 4-Me | Me | 3-Me-Ph | H | 138 |
| 35 | 4-PhO | Me | 2-Me-Ph | H | 120 |
| 36 | 4-F | Me | 4-Cl-Ph | H | 142 |
| 37 | - | Me | 4-F-Ph | H | 157 |
| 38 | - | Me | 2,4-di(F)-Ph | H | 166 |
| 39 | - | Me | 4-Me-Ph | H | 116 |
| 40 | 4-F | Me | 2-F-Ph | H | miel |
| 41 | 4-Me | Me | 2-F-Ph | H | 90 |
| 42 | 4-Cl | Me | 2-F-Ph | H | miel |

| | | | | | |
|---|---|---|---|---|---|
| 43 | 4-F | Me | 2-Cl-Ph | H | miel |
| 44 | - | Me | 2,3-di(Me)-Ph | H | 140 |
| 45 | - | Me | 3-Cl-2-Pyridyl | H | 140 |
| 46 | - | Me | 3-Me-Ph | H | 54 |
| 47 | - | Me | 2-F-Ph | H | 136 |
| 48 | - | Me | 3-Cl-Ph | H | 93 |
| 49 | 4-F | Me | 3-Me-2-Pyridyl | H | 126 |
| 50 | 4-F | Me | 3-F-Ph | H | 120 |
| 51 | 4-Cl | Me | 3-F-Ph | H | 125 |
| 52 | 3-Cl | Me | Ph | H | 155 |
| 53 | 3-Cl | Me | 3-Me-Ph | H | 100 |
| 54 | 3-Cl | Me | 3-Cl-Ph | H | 105 |
| 55 | 3-Cl | Me | 3-F-Ph | H | 135 |
| 56 | 3-Cl | Me | 2-Me-Ph | H | 116 |
| 57 | 3-F | Me | Ph | H | 134 |
| 58 | 3-F | Me | 3-F-Ph | H | 115 |
| 59 | - | Me | Ph | Ac | 121 |
| 60 | 3-F | Me | 3-Cl-Ph | H | 90 |
| 61 | 3-Me | Me | Ph | H | 98 |
| 62 | 3-Me | Me | 3-F-Ph | H | 118 |
| 63 | 4-PhO | Me | 2-Pyridyl | H | 146 |

| | | | | | |
|---|---|---|---|---|---|
| 64 | 2,4-diF | Me | Ph | H | 163 |
| 65 | 2,4-diF | Me | 3-F-Ph | H | 162 |
| 66 | 4-PhO | Me | Ph | H | 114 |
| 67 | 4-PhO | Me | 3-F-Ph | H | 56 |
| 68 | 2-Cl | Me | Ph | H | 214 |
| 69 | - | Me | 4-PhO-Ph | H | 118 |
| 70 | 2-Cl | Me | 3-F-Ph | H | 201 |
| 71 | 2-F | Me | Ph | H | 172 |
| 72 | 2-F | Me | 3-F-Ph | H | 151 |
| 73 | 2,4-diF | Me | 3-Cl-Ph | H | 130 |
| 74 | 2-F | Me | 3-Cl-Ph | H | 151 |
| 75 | 4-iPr | Me | Ph | H | 132 |
| 76 | 4-PhO | Me | 3-Me-2-Pyridyl | H | 142 |
| 77 | 4-iPr | Me | 3-F-Ph | H | 128 |
| 78 | 3-Me | Me | 3-Cl-Ph | H | 115 |
| 79 | 4-iPr | Me | 3-Cl-Ph | H | 139 |
| 80 | 4-Br | Me | Ph | H | 138 |
| 81 | 4-$NO_2$ | Me | Ph | H | 143 |
| 82 | 3-PhO | Me | Ph | H | miel |
| 83 | 2,4-di(F)-4-PhO | Me | 3-F-Ph | H | 71 |
| 84 | 2,4-di(F)-4-PhO | Me | Ph | H | 76 |
| 85 | 4-F-4-PhO | Me | Ph | H | 91 |

Composé n° 7 :

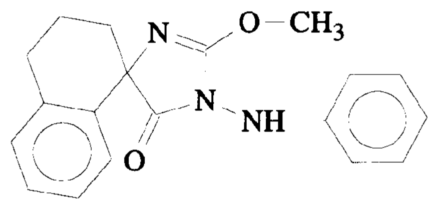

PF = 73°C

Composé n° 10 :

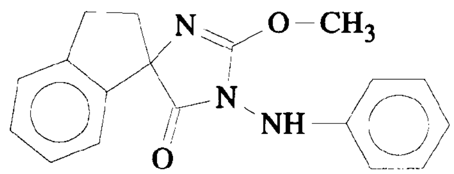

PF = 86°C

Composé n° 22 :

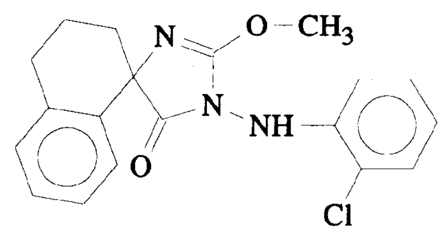

PF = 71°C

Composé n° 86 :

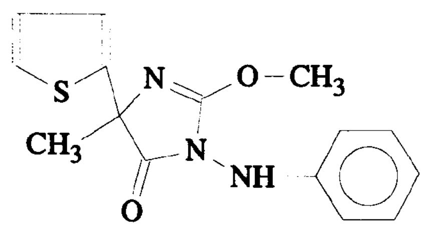

PF = 98°C

<u>Exemple 2</u> : Test in vivo sur *Plasmopara viticola* (mildiou de la vigne) :

On prépare, par broyage fin, une suspension aqueuse de la matière active à tester ayant la composition suivante:

- matière active: 60 mg
- agent tensioactif Tween 80, (oléate de dérivé polyoxyéthyléné du sorbitan) dilué à 10% dans l'eau: 0,3 ml
- on complète à 60 ml d'eau.

Cette suspension aqueuse est ensuite diluée par de l'eau pour obtenir la concentration désirée en matière active.

Des boutures de vigne (Vitis vinifera), variété Chardonnay, sont cultivées dans des godets. Lorsque ces plants sont âgés de 2 mois (stade 8 à 10 feuilles, hauteur de 10 à 15 cm), ils sont traités par pulvérisation au

moyen de la suspension aqueuse ci-dessus.

Des plants, utilisés comme témoins sont traités par une solution aqueuse ne contenant pas la matière active.

Après séchage pendant 24 heures, on contamine chaque plant par pulvérisation d'une suspension aqueuse de spores de *Plasmopara viticola* obtenue à partir d' une culture de 4-5 jours, mise ensuite en suspension à raison de 100 000 unités par $cm^3$.

Les plants contaminés sont ensuite mis en incubation pendant deux jours à 18°C environ, en atmosphère saturée d'humidité puis pendant 5 jours à 20-22°C environ sous 90-100% d'humidité relative.

La lecture se fait 7 jours après la contamination, en comparaison avec les plants témoins.

Dans ces conditions, on observe, à la dose de 1 g/l, une protection bonne (au moins 75%) ou totale avec les composés suivants : 1, 3 à 21, 23 à 25, 27 à 81, 83, 84, 86.

<u>Exemple 3</u> : Test in vivo sur *Puccinia recondita* (rouille du blé) :

On prépare, par broyage fin, une suspension aqueuse de la matière active à tester ayant la composition suivante:

- matière active: 60 mg
- agent tensioactif Tween 80, (oléate de dérivé polyoxyéthyléné du sorbitan) dilué à 10% dans l'eau: 0,3 ml
- on complète à 60 ml d'eau.

Cette suspension aqueuse est ensuite diluée par de l'eau pour obtenir la concentration désirée en matière active.

Du blé, en godets, semé sur un substrat tourbe terre pouzzolane 50/50, est traité au stade 10 cm de hauteur par pulvérisation de la suspension aqueuse ci dessus.

Au bout de 24 heures, une suspension aqueuse de spores (100000 sp/$cm^3$) est pulvérisée sur le blé; cette suspension a été obtenue à partir de plants contaminés. On place ensuite le blé pendant 24 heures en cellule d'incubation à environ 20°C et à 100% d'humidité relative, puis pendant 7 à 14 jours à 60% d'humidité relative.

Le contrôle de l'état des plants se fait entre le 8ème et le 15ème jour après la contamination, par comparaison avec un témoin non traité.

Dans ces conditions, on observe, à la dose de 1 g/l, une protection bonne (au moins 75%) ou totale avec les composés suivants 1, 4 à 10, 14, 15, 19 à 21, 23, 25, 27 à 29, 31 à 33, 36, 37, 39, 40 à 48, 50 à 55, 57, 58, 60, 61, 63 à 67, 69, 71 à 80, 82 à 84, 86.

<u>Exemple 4</u> : Test in vivo sur *Phytophthora infestans* (mildiou de la tomate) :

On prépare, par broyage fin, une suspension aqueuse de la matière active à tester ayant la composition suivante:

- matière active: 60 mg
- agent tensioactif Tween 80, (oléate de dérivé polyoxyéthyléné du sorbitan) dilué à 10% dans l'eau: 0,3 ml
- on complète à 60 ml d'eau.

Cette suspension aqueuse est ensuite diluée par de l'eau pour obtenir la concentration désirée en matière active.

Des plants de tomate (variété Marmande) sont cultivés dans des godets. Lorsque ces plants sont agés d'un mois (stade 5 à 6 feuilles, hauteur 12 à 15 cm), ils sont traités par pulvérisation de la suspension aqueuse ci dessus et à diverses concentrations du composé à tester.

Au bout de 24 heures, on contamine chaque plant par pulvérisation au moyen d'une suspension aqueuse de spores (30000 sp/$cm^3$) de Phytophthora infestans.

Après cette contamination, les plants de tomate sont mis en incubation pendant 7 jours à 20°C environ en atmosphère saturée d'humidité.

Sept jours après la contamination, on compare les résultats obtenus dans le cas des plants traités par la matière active à tester à ceux obtenus dans le cas des plants utilisés comme témoins. Dans ces conditions, on observe, à la dose de 1 g/l, une protection bonne (au moins 75%) ou totale avec les composés suivants: 1, 4 à 10, 15, 18 à 21, 23, 28, 29, 31 à 33, 36, 37, 39, 42 à 44, 46 à 55, 57, 58, 60 à 67, 72 à 74, 81, 84, 86.

<u>Exemple 5</u> : Test in vivo sur *Pyricularia oryzae* (piriculariose du riz) :

On prépare, par broyage fin, une suspension aqueuse de la matière active à tester ayant la composition

suivante :

- matière active : 60 mg
- agent tensioactif (oléate de dérivé polyoxyéthyléné du sorbitan) dilué à 10% dans l'eau : 0,3 ml
- on complète à 60 ml d'eau.

Cette suspension aqueuse est ensuite diluée par de l'eau pour obtenir la concentration désirée en matière active.

Du riz, semé en godets dans un mélange 50/50 de tourbe enrichie et de pouzzolane, est traité au stade d'environ 10 cm de hauteur ( ce qui correspond au stade 2-3 feuilles) par pulvérisation de la suspension aqueuse ci dessus.

Au bout de 24 heures, on applique sur les feuilles une suspension aqueuse de spores de Pyricularia oryzae, obtenue à partir d' une culture de 15 jours, mise ensuite en suspension à raison de 100 000 unités par $cm^3$.

Les plants de riz sont placés pendant 24 heures en incubation ( 25°C, 100% d'humidité relative) , puis mis en cellule d'observation, dans les mêmes conditions, pendant 5 jours.

La lecture se fait 6 jours après la contamination.

Dans ces conditions, on observe, à la dose de 1 g/l, une protection bonne (au moins 75%) ou totale avec les composés suivants : 1, 4 à 7, 17, 19, 20, 23, 25, 29, 31 à 33, 35, 40 à 43, 46, 47, 50, 52, 54, 55, 57, 59, 61, 63 à 67, 71, 72, 74, 78, 82, 84, 85.

Exemple 6 : Test in vivo sur *Septoria tritici* (septoriose du blé) :

Une suspension aqueuse, de concentration 1 g/l, de la matière active testée est obtenue par broyage de 60 mg de celle-ci dans le mélange suivant :

- acétone : 5 ml
- agent tensioactif(oléate de dérivé polyoxyéthyléné du sorbitan) dilué à 10 % : 0,3ml
  puis le volume est ajusté à 60 ml avec de l'eau.

Cette suspension aqueuse est ensuite diluée par de l'eau pour obtenir la concentration désirée en matière active.

Des plants de blé (variété Darius), semés sur un substrat tourbe-pouzzolane 50/50 et cultivés en serre à température 10-12 °C, sont traités au stade 1 feuille (taille de 10 cm environ) par pulvérisation de la suspension de matière active décrite ci-dessus.

Des plants, utilisés comme témoins, sont traités par pulvérisation d'une solution aqueuse ne contenant pas la matière active.

24 heures après traitement, les plants sont contaminés par pulvérisation d'une suspension aqueuse de spores (500 000sp/ml) récoltés sur une culture âgée de 7 jours.

Après contamination, les plants sont placés à 18°C en atmosphère humide. La notation est effectuée 20 jours après la contamination en comparaison avec les plants témoins.

Dans ces conditions, on observe à la dose de 1 g/l, une bonne protection (au moins 75 %) ou totale avec les composés : 1, 4 à 6, 9, 19, 20, 23, 32, 33, 35 à 37, 40, 42 à 45, 47, 48, 50, 52, 54, 55, 57, 58, 60, 62 à 64, 66, 67, 71 à 80, 83, 84, 86.

Ces résultats montrent clairement les bonnes propriétés fongicides des dérivés selon l'invention contre les maladies fongiques des plantes dues à des champignons appartenant aux familles les plus diverses telles que les Phycomycètes, les Basidiomycètes, les Ascomycètes, les Adelomycètes ou Fungi imperfecti, en particulier le mildiou de la vigne, le mildiou de la tomate, les rouilles et les septorioses du blé et aussi la piriculariose du riz.

Pour leur emploi pratique, les composés selon l'invention sont rarement utilisés seuls. Le plus souvent ces composés font partie de compositions. Ces compositions, utilisables comme agents fongicides, contiennent comme matière active un composé selon l'invention tel que décrit précédemment en mélange avec les supports solides ou liquides, acceptables en agriculture et/ou les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloides protecteurs, des adhésifs, des épaississants, des agents thixotropes, des agents de pénétration, des stabilisants, des séquestrants, etc... Plus généralement les composés utilisés dans l'invention peuvent être combinés à tous les additifs solides ou liquides correspondant aux techniques habituelles de la mise en formulation.

Sauf indication contraire, les pourcentages indiqués dans la présente description sont exprimés en poids.

D'une façon générale, les compositions selon l'invention contiennent habituellement de 0,05 à 95 % environ (en poids) d'un composé selon l'invention (appelé par la suite matière active), un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol etc...).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de très larges limites, allant de 0,05 % à 95 % (en poids). Leur teneur en agent tensio-actif est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés. notamment ceux obtenus par extrusion, par compactage, par imprégnation d'un support granulé, par granulation à partir d'une poudre (la teneur en composé dans ces granulés étant entre 0,5 et 80 % pour ces derniers cas), les comprimés ou tablettes effervescents.

Les composés de formule (I) peuvent encore être utilisés sous forme de poudres pour poudrage; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes, les gels.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnables :

Exemple CE 1 :

- matière active 400 g/l
- dodécylbenzène sulfonate alcalin 24 g/l
- nonylphénol oxyéthylé à 10 molécules d'oxyde d'éthylène 16 g/l
- cyclohexanone 200 g/l
- solvant aromatique q.s.p. 1 litre

Selon une autre formule de concentré émulsionnable, on utilise :

Exemple CE 2 :

- matière active 250 g
- huile végétale époxydée 25 g
- mélange de sulfonate d'alcoylaryle et d'éther de polyglycol et d'alcools gras 100 g
- diméthylformamide 50 g
- xylène 575 g

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

Exemple SC 1 :

- matière active 500 g
- phosphate de tristyrylphénol polyéthoxylé 50 g
- alkylphénol polyéthoxylé 50 g
- polycarboxylate de sodium 20 g
- éthylène glycol 50 g
- huile organopolysiloxanique (antimousse) 1 g
- polysaccharide 1,5 g
- eau 316,5 g

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0,1 à 10 % d'un ou plusieurs stabilisants et/ou autres additifs, comme des agents de pénétration, des adhésifs, ou des agents antimottants, colorants, etc... Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables trés avantageusement en particulier pour l'application sur les feuilles des végétaux. A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

Exemple PM 1 :

- matière active 50%
- alcool gras éthoxylé (agent mouillant) 2,5%
- phényléthylphénol éthoxylé (agent dispersant) 5 %
- craie (support inerte) 42,5%

Exemple PM 2 :

- matière active 10%
- alcool synthétique oxo de type ramifié, en C13 éthoxylé par 8 à 10 oxyde d'éthylène (agent mouillant) 0,75%
- lignosulfonate de calcium neutre (agent dispersant) 12%
- carbonate de calcium (charge inerte) q.s.p. 100 %

Exemple PM 3 :

Cette poudre mouillable contient les mêmes ingrédients que dans l'exemple précédent, dans les proportions ci-après:

- matière active 75 %
- agent mouillant 1,50%
- agent dispersant 8 %
- carbonate de calcium (charge inerte) q.s.p. 100%

Exemple PM 4 :

- matière active 90%
- alcool gras éthoxylé (agent mouillant) 4%
- phényléthylphénol éthoxylé (agent dispersant) 6%

Exemple PM 5 :

- matière active 50%
- mélange de tensio-actifs anioniques et non ioniques (agent mouillant) 2,5%
- lignosulfonate de sodium (agent dispersant) 5 %
- argile kaolinique (support inerte) 42,5%

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple GD1 : Granulés dispersibles

Dans un mélangeur, on mélange 90 % en poids de matière active et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple GD2: Granulés dispersibles

Dans un mélangeur, on mélange les constituants suivants:

- matière active 75%
- agent mouillant (alkylnaphtalène sulfonate de sodium) 2%
- agent dispersant (polynaphtalène sulfonate de sodium) 8%
- charge inerte insoluble dans l'eau (kaolin) 15%

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

En ce qui concerne les compositions adaptées au stockage et au transport, elles contiennent plus avantageusement de 0,5 à 95 % (en poids) de substance active.

L'invention a également pour objet l'utilisation des composés selon l'invention pour la lutte contre les maladies fongiques des plantes par traitement préventif ou curatif de ces dernières ou de leur lieu de croissance.

Ils s'appliquent avantageusement à des doses de 0,005 à 5 kg/ha, et plus spécifiquement de 0,01 à 1 kg/ha.

## Revendications

**1)** Composés 2-alkoxy-2-imidazoline-5-ones de formule générale (I)

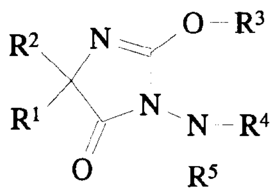

dans laquelle :

- $R^1$ représente un radical aryl comprenant phényl, naphtyl, thiényl, furyl, pyridyl, benzothiényl, benzofuryl, quinolinyl, isoquinolinyl, ou méthylène dioxyphényl, chacun de ces radicaux étant éventuellement substitué par 1 à 3 groupements choisis parmi les significations de $R^6$ ;
- $R^2$ représente un radical alkyl ou haloalkyl de 1 à 3 atomes de carbone,
- $R^1$ et $R^2$ pouvant en outre former, avec le carbone auquel ils sont liés sur le cycle, un carbocycle ou un hétérocycle ayant de 5 à 7 atomes, ces cycles pouvant être fusionnés avec un phényl, chacun de ces radicaux étant éventuellement substitué par 1 à 3 groupes choisis parmi les significations de $R^6$ ;
- $R^3$ représente un groupe alkyl ou haloalkyl de 1 à 3 atomes de carbone ;
- $R^4$ représente un radical aryl, comprenant phényl, naphtyl, pyridyl, pyrimidyl, pyridazinyl, pyrazinyl, benzothiényl, benzofuryl, quinolinyl, isoquinolinyl, ou méthylène dioxyphényl, chacun de ces radicaux étant éventuellement substitué par 1 à 3 groupements choisis parmi les significations de $R^6$ ;
- $R^5$ représente un atome d'hydrogène ou un radical formyl, acyl de 2 à 6 atomes de carbone, aroyl, alkoxycarbonyl de 2 à 6 atomes de carbone, aryloxycarbonyl, alkylsulfonyl, arylsulfonyl;
- $R^6$ représente:
  - un atome d'halogène ou
  - un radical alkyl, haloalkyl, cyanoalkyl, alkoxy, haloalkoxy, cyanoalkoxy, alkylthio, haloalkylthio, cyanoalkylthio ou alkylsulfonyl de 1 à 6 atomes de carbone ou
  - un radical cycloalkyl, halocycloalkyl, alcényl, alcynyl, alcényloxy, alcynyloxy, alcénylthio, alcynylthio de 3 à 6 atomes de carbone ou
  - le groupe nitro ou cyano ou thiocyanato ou
  - un radical amino éventuellement mono ou disubstitué par un radical alkyl ou acyl de 1 à 6 atomes de carbone ou alkoxycarbonyl de 2 à 6 atomes de carbone
  - un radical phényl, phénoxy, phénylthio, phénylsulfonyl ou pyridyloxy, ces radicaux étant éventuellement substitués par un ou plusieurs des groupes suivants :
    - un atome d'halogène ou
    - un radical alkyl, haloalkyl, cyanoalkyl, alkoxy, haloalkoxy, alkylthio, haloalkylthio ou alkylsulfonyl de 1 à 6 atomes de carbone ou
    - un radical cycloalkyl, halocycloalkyl, alcényl, alcynyl, alcényloxy, alcynyloxy, alcénylthio, alcynylthio de 3 à 6 atomes de carbone ou
    - le groupe nitro ou cyano ou thiocyanato ou
    - un radical amino éventuellement mono ou disubstitué par un radical alkyl ou acyl de 1 à 6 atomes de carbone ou alkoxycarbonyl de 2 à 6 atomes de carbone
    - un radical phényl, phénoxy, phénylthio, phénylsulfonyl ou pyridyloxy,

et leurs formes salifiées.

**2)** Composés selon la revendication 1 caractérisés en ce qu'ils répondent à la formule générale II :

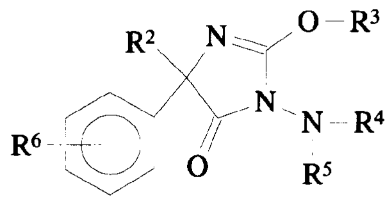

II

dans laquelle $R^1$ à $R^6$ ont les mêmes significations que dans la formule I.

**3)** Procédé de préparation des composés selon la revendication 1, caractérisé en ce qu'on fait réagir les 2-alkylthio-2-imidazoline-5-ones de formule (III) avec un alcool $R^3OH$ en présence de base forte, selon le schéma :

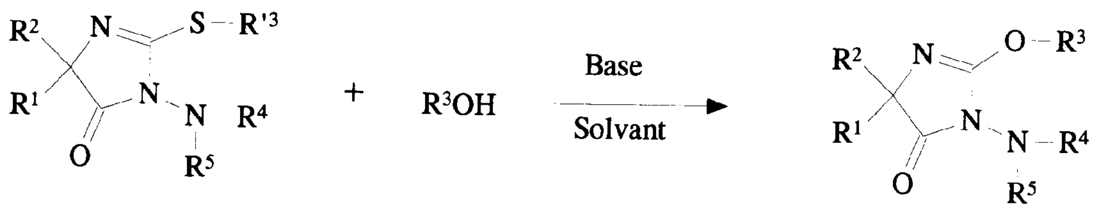


III

I

$R^1$, $R^2$, $R^3$ et $R^4$ ayant la même signification que pour la formule I et $R'^3$ représentant un groupe alkyl de 1 à 3 atomes de carbone.

**4)** Compositions fongicides comprenant, en association avec un ou des supports solides ou liquides acceptables en agriculture et/ou des agents tensioactifs également acceptables en agriculture, une (ou plusieurs) matière active qui est un composé de formule I selon la revendication 1.

**5)** Compositions fongicides comprenant, en association avec un ou des supports solides ou liquides acceptables en agriculture et/ou des agents tensioactifs également acceptables en agriculture, une (ou plusieurs) matière active qui est un composé de formule II selon la revendication 2.

**6)** Compositions fongicides selon l'une des revendications 4 ou 5, caractérisées en ce qu'elles contiennent de 0,5 % à 95 % en poids de composés selon l'une des revendications 1 ou 2.

**7)** Procédé de traitement des cultures atteintes ou susceptibles d'être atteintes par les maladies fongiques caractérisé en ce que l'on applique, de façon préventive ou curative, une quantité efficace d'un composé selon l'une des revendications 1 ou 2 ou d'une composition selon les revendications 4 à 6.

**8)** Procédé de traitement selon la revendication 7, caractérisé en ce que la dose efficace est comprise entre 0,005 et 5 kg/ha.

**9)** Procédé de traitement selon la revendication 8, caractérisé en ce que la dose efficace est comprise entre 0,01 et 1 kg/ha.

Office européen des brevets

# RAPPORT DE RECHERCHE EUROPEENNE

Numero de la demande

EP 93 42 0462
PAGE1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | GB-A-967 166 (IMPERIAL CHEMICAL INDUSTRIES LTD.)<br>* revendications *<br>--- | 1-9 | C07D233/80<br>C07D235/02<br>C07D401/12<br>A01N43/50<br>C07D409/04 |
| A | GB-A-967 167 (IMPERIAL CHEMICAL INDUSTRIES LTD.)<br>* exemples 1,2,9,10 *<br>--- | 1-9 | |
| D,P,<br>A | EP-A-0 551 048 (RHONE-POULENC AGROCHIMIE)<br>* le document en entier *<br>--- | 1-9 | |
| A | J. INDIAN CHEM. SOC.<br>vol. 66, no. 11, 1989,<br>pages 813 - 814<br>B. MISHRA ET AL.<br>--- | 1-9 | |
| A | CHEMICAL ABSTRACTS, vol. 116, no. 25,<br>22 Juin 1992, Columbus, Ohio, US;<br>abstract no. 255536k,<br>AZZA M. KADRY ET AL.<br>* abrégé *<br>& BULL. FAC. PHARM.<br>vol. 29, no. 3, 1991,<br>pages 21 - 25<br>--- | 1-9 | DOMAINES TECHNIQUES RECHERCHES (Int. Cl.5 )<br>C07D<br>A01N |
| A | CHEMICAL ABSTRACTS, vol. 115, no. 5,<br>5 Août 1991, Columbus, Ohio, US;<br>abstract no. 49535k,<br>M.A.H. ISMAIL ET AL.<br>* abrégé *<br>& BULL. FAC. PHARM.<br>vol. 28, no. 3, 1990,<br>pages 15 - 18<br>--- | 1-9 | |
| A | INDIAN J. CHEM. SECT. B<br>vol. 20B, no. 12, 1981,<br>pages 1093 - 1094<br>M.Z. BADR ET AL.<br>--- | 1-9 | |

Le présent rapport a été établi pour toutes les revendications

| Lieu de la recherche | Date d'achèvement de la recherche | Examinateur |
|---|---|---|
| BERLIN | 04 JANVIER 1994 | FRELON D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 93 42 0462
PAGE2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | INDIAN J. PHARM. SCI.<br>vol. 54, no. 3, 1992,<br>pages 119 - 121<br>B.H. TRIVEDI ET AL.<br>----- | 1-9 | |
| | | | DOMAINES TECHNIQUES RECHERCHES (Int. Cl.5 ) |

Le présent rapport a été établi pour toutes les revendications

| Lieu de la recherche | Date d'achèvement de la recherche | Examinateur |
|---|---|---|
| BERLIN | 04 JANVIER 1994 | FRELON D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)